Europäisches Patentamt

European Patent Office

Office européen des brevets

(11) Publication number: 0 452 780 A2

# EUROPEAN PATENT APPLICATION

(21) Application number: 91105630.7

(22) Date of filing: 09.04.91

(51) Int. Cl.5: A61L 2/20

(30) Priority: 17.04.90 US 510317

(43) Date of publication of application:
23.10.91 Bulletin 91/43

(84) Designated Contracting States:
AT BE CH DE DK ES FR GB GR IT LI NL SE

(71) Applicant: ABBOTT LABORATORIES
CHAD-0377, AP6D/2, One Abbott Park Road
Abbott Park, Illinois 60064-3500(US)

(72) Inventor: Kruger, Robert J.
1225 South Patton Avenue
Arlington Heights, Illinois 60005(US)
Inventor: Mayoral, Joaquin
213 Midlothian
Mundelein, Illinois 60060(US)

(74) Representative: Modiano, Guido et al
MODIANO, JOSIF, PISANTY & STAUB
Modiano & Associati Via Meravigli, 16
I-20123 Milano(IT)

(54) Method for sterilizing an enclosure with noncondensing hydrogen peroxide-containing gas.

(57) A method for sterilization of an enclosure using a dry hydrogen peroxide-containing gas and a fill apparatus useful therefore are disclosed. Sterilization is effected in a relatively short time period while non-condensing conditions for the sterilizing gas are maintained.

FIG-1

EP 0 452 780 A2

Technical Field

This invention relates to sterilization systems and, in particular, to sterilization systems that utilize gaseous hydrogen peroxide.

Background of the Invention

Hydrogen peroxide has long been used as an antiseptic or disinfectant. However, hydrogen peroxide is relatively unstable and decomposes rapidly. Therefore, its widespread use has been hampered by the difficulty of storing solutions of hydrogen peroxide.

Several processes for sterilizing articles have utilized the technique of vaporizing the hydrogen peroxide and introducing the vapor onto a surface to be sterilized. The vapor is generally a multicomponent admixture as the hydrogen peroxide is generally mixed with water prior to vaporization.

In U.S. Patent No. 4,642,165 to Bier, hydrogen peroxide ($H_2O_2$) mixed with water is vaporized and drawn into a chamber by vacuum. Bier was attempting to resolve the problem of the water vaporizing more readily and in greater quantity than the hydrogen peroxide due to the relatively low boiling point of the former. The more ready vaporization of water caused the water vapor to reach the object to be sterilized first and, condensing thereon, prevented the hydrogen peroxide vapor from fully contacting the object. The vaporization in Bier is done incrementally to ensure that the relative amounts of peroxide and water vapor present in the liquid are approximately maintained as the vaporization proceeds so that both vapor components reach the object to be sterilized at the same time. The hydrogen peroxide solution is vaporized using a heating element. Control over other environmental factors influencing the vapor formation and subsequent condensation is not exerted. Bier utilizes a hydrogen peroxide solution that is approximately twenty to fifty percent by weight hydrogen peroxide. The pressure in the sterilization chamber which draws in the vapor is about 10 mmHg or less.

U.S. Patent No. 4,169,124 to Forstrom et al., teaches a cold gas sterilization process at a temperature below $80^\circ$ C using a low concentration of hydrogen peroxide (<75mg/L). Negative pressure is again utilized to draw the vapor into the chamber, but the reported pressure is preferably greater than 15 inches of Hg. Sixty seconds to one hour is said to be necessary to achieve the desired degree of sterility using this process. According to this patent, it requires six to twenty-four hours for the sporicidal effect of this method to be realized.

U.S. Patent No. 4,424,189 to Hick teaches spraying hydrogen peroxide onto a heating element. The formation of free oxygen resulting from flash vaporization is taught as necessary to achieve an antiseptic effect. The object to be sterilized is wetted with the atomized hydrogen peroxide vapor.

None of the forgoing processes using hydrogen peroxide vapor for sterilization achieves a high degree of sporicidal effect in a relatively short amount of time of the order of about 10 seconds or less. However, the United States Food and Drug Administration (FDA) is currently recommending that all medical and surgical products be sterilized to a very low probability of survival for spores, which are among the most difficult microorganisms to kill. The recommended degree of kill is that providing a sporicidal efficacy sufficient to assure a microbial survival probability of $10^{-6}$. That is, to provide an assurance that there is less than one chance in one million that a viable microorganism is present in the sterilized article or enclosure. This sterility level can also be expressed as the negative logarithm to base 10 of microorganism survival probability, or "$\log_{10}$ kill." Thus, a survival probability of $10^{-6}$ can also be concisely stated as 6 $\log_{10}$ kill.

For a general discussion of sterilization and techniques therefor, see The United States Pharmacopeia XXII, ch. 1211, pp. 1705 et seq., Atkinson et al., Biochemical Engineering and Biotechnology Handbook, Stockton Press, New York, N.Y. (1983), pp. 875-886, and Demain et al., Manual of Industrial Microbiology and Biotechnology, American Society for Microbiology, Washington, D.C. (1986), pp. 345-362.

Summary of the Invention

The present method provides efficient sterilization of an enclosure, or the contents thereof, at a subatmospheric pressure in a matter of seconds, or less. The specific conditions in the enclosure are determined by the degree of spore kill desired. Sterilization is achieved by maintaining a hydrogen peroxide gas under non-condensing conditions. That is, the sterilizing gas present is maintained above its dew point temperature at the existing subatmospheric pressure while in the enclosure to be sterilized.

The method of this invention contemplates use, under subatmospheric pressure conditions, of a dry hydrogen-peroxide containing gas, i.e., a moistureless gas that will not condense upon introduction into the enclosure to be sterilized. This enclosure is first isolated from its surroundings and then evacuated to a predetermined residual pressure. An aliquot of the dry sterilizing gas is introduced into the enclosure and the temperature therein is maintained at conditions which ensure that condensation of the gas will not take place. After sterilization to the desired degree, the hydrogen peroxide gas is evacuated from the enclosure. The enclosure is then sealed. An optional purging step, using a sterile gas, can also be carried out if it is desired to remove residuals from the enclosure prior to sealing it.

The present process has the advantage of achieving a high degree of sporicidal effect in a relatively short amount of time. It only takes seconds, or less, to sterilize a particular enclosure or the contents thereof. Thus, rapid sterilization at fluid filling line speeds can be readily achieved.

Brief Description of the Drawings

FIGURE 1 is a schematic representation of the present sterilization process;

FIGURE 2 is a perspective view of a connector means for an enclosure to be sterilized as contemplated by the present process;

FIGURE 3 is a sectional elevation view of the connector shown in FIGURE 2 as connected to both a sterilizing gas source and the enclosure to be sterilized;

FIGURE 4 is a three-axis graphical presentation illustrating the inter-relationship of sterilization process parameters for a desired spore kill; and

FIGURE 5 is a graphical presentation illustrating the inter-relationship among change in residual pressure, temperature, and concentration of hydrogen peroxide present in the introduced sterilizing gas during the sterilization process.

Detailed Description of the Preferred Embodiments

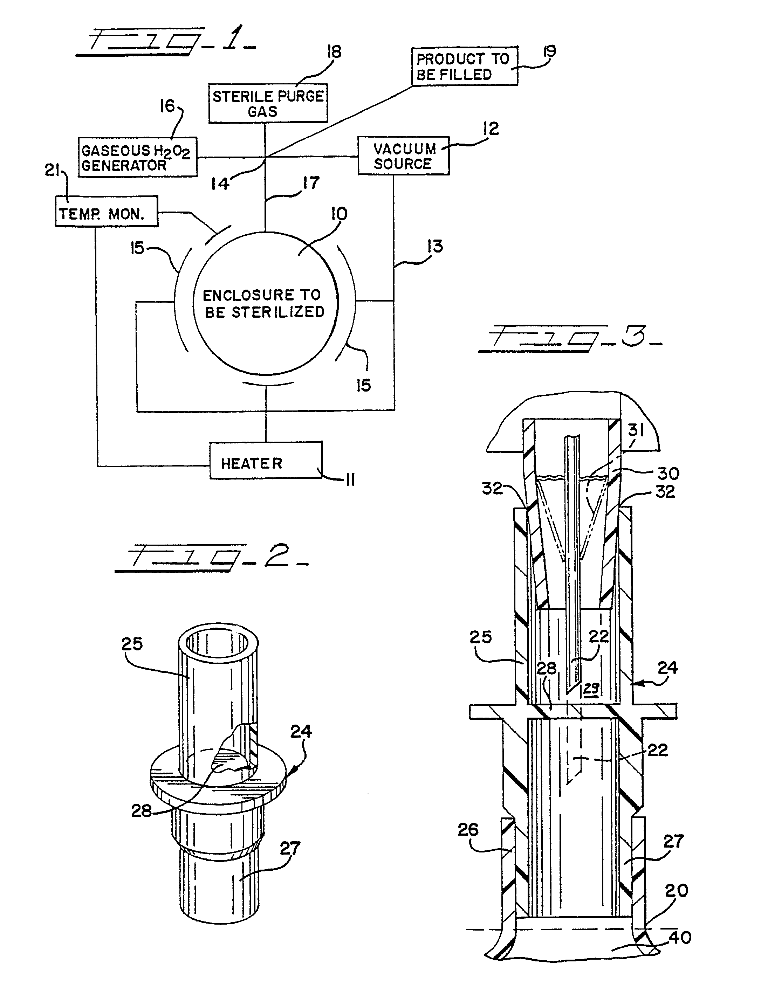

The sterilization system for practicing the method of this invention is schematically illustrated in FIGURE 1. Hydrogen peroxide source 16, vacuum source 12, sterile purge gas source 18 and product source 19 all communicate with a common manifold equipped with a valving means at 14 that controls access to the enclosure 10 to be sterilized via passageway 17. In instances where the enclosure 10 has flexible walls that may collapse when vacuum is drawn, vacuum source 12 also communicates with vacuum - energized gripper means 15 via vacuum line 13 which gripper means hold the walls of enclosure 10 open while vacuum is drawn. Heater means 11 in operative association with the temperature monitor 21 are provided to maintain the enclosure to be sterilized at the desired temperature during the sterilization process.

For sterilization purposes, the degree of vacuum drawn, i.e., the residual pressure within the enclosure to be sterilized, depends on the rate and severity of desired kill, as well as on the concentration of hydrogen peroxide in the solution to be vaporized to generate the sterilizing gas, the temperature of that solution, and similar factors. These operating conditions are determined prior to sterilization of the enclosure and any items placed therein, and are monitored during sterilization. Usually the enclosure 10 is evacuated to a residual pressure of no more than about 50 mmHg and, preferably, to a residual pressure of no more than about 20 mmHg. The more preferred residual pressure is about 15 mmHg. The residual pressure also provides a driving force for the introduction of the sterilizing gas into the enclosure to be sterilized as well as for expediting purge cycles after sterilization as will be discussed in detail hereinbelow.

When the desired residual pressure is established in the enclosure 10, an aliquot of gaseous hydrogen peroxide-containing sterilizing gas is introduced into the enclosure 10 in a single step. This is accomplished by use of a manifold valve means 14. The process can be automated by providing a multiport valve at 14 which first provides fluid communication between the vacuum source 12 and the enclosure 10, enabling a vacuum to be drawn thereon. In the latter case, when the desired residual pressure is reached, the valve automatically turns, next providing fluid communication between the enclosure 10 and the sterilizing gas source 16. Prior to introduction into the enclosure 10, the hydrogen peroxide-containing sterilizing gas is above its dew point temperature at the existing pressure.

As the sterilizing gas is introduced into the enclosure 10, pressure and temperature conditions are maintained therein to ensure that no condensation of the sterilizing gas takes place. To that end, enclosure 10 can be preheated, or the sterilizing gas may be sufficiently superheated to provide the necessary heat transfer to the enclosure 10. The sterilizing gas, and thus the hydrogen peroxide gas constituent thereof, remain above their respective dew point temperatures at the existing pressure while in the enclosure 10. The concentration of the hydrogen peroxide in the sterilizing gas is sufficient to ensure that this gas is sporicidal to a predetermined degree. If the heat delivered by the sterilizing gas is insufficient to maintain the temperature of the surfaces contacted by the sterilizing gas in the enclosure above the dew point temperatures, the enclosure, or an object placed therein to be sterilized, can be heated during sterilization by any suitable means, e.g., by radiant heat, by microwave energy in the case of conductive materials, by conductive heat transfer, or by similar expedients.

The concentration of hydrogen peroxide gas in the introduced sterilizing gas can be in the range of about 1.5 mg/l to about 150 mg/l, preferably about 5 mg/l to about 100 mg/1, depending upon the initial or residual pressure in the enclosure 10 when the sterilizing gas is introduced therein. At a relatively higher residual pressure the time required to reach the desired effective spore kill is longer.

A sterilization temperature range of 20°C to 120°C is selected to ensure that the gaseous hydrogen peroxide/water admixture present remains gaseous within the other parameters (i.e., pressure, volume, concentration) of the process. The term "dry", as used herein and in the appended claims to characterize the sterilizing gas, means that the sterilizing gas is moistureless and mist-free. The sterilizing gas may contain non-condensable water vapor, however.

The enclosure 10 to be sterilized, and any contents thereof are heated to approximately the same temperature as the sterilizing gas to avoid condensation of the gas on cooler surfaces. Obviously, the higher the temperature, the less likely the gas will condense assuming all other parameters remain constant. When the sterilization temperature is within the temperature range of 20°C to 120°C, residual pressure is a more significant parameter than time, as will be discussed in greater detail hereinbelow.

The dry sterilizing gas is held within the enclosure 10 for a time period sufficient to effect the desired degree of kill of spores or other bacteria that might be present therein. As spores are the hardiest of the microorganisms that might be present in the enclosure 10, the killing of a significant portion of the spore population results in the kill of essentially all other microorganisms as well. The time for which the hydrogen peroxide sterilizing gas is held in the enclosure 10 in the presence of the microorganisms to be eradicated can vary. To achieve 6 $\log_{10}$ kill the time is generally less than about one second, and can be as short as a fraction thereof. The specific time in which the desired degree of spore kill is achieved is dependent upon the process parameters (i.e., temperature, pressure, concentration), but is relatively short. Using the parameters at which the hydrogen peroxide is at its dew point temperature as point zero, the greater the deviation of these parameters above point zero, the less-the time necessary to achieve the desired degree of kill. For a lower degree of kill, e.g., 5 $\log_{10}$ kill, milder conditions are sufficient, but noncondensing conditions must be maintained. At the preferred sterilization temperatures and pressures, once the desired conditions are reached within the enclosure to be sterilized, the sterilization itself takes place virtually instantaneously, and processing time becomes primarily a function of available process equipment.

After the desired degree of spore kill, valve 14 allows the now sterilized enclosure to be purged, if desired, of any remaining hydrogen peroxide gas and any residuals resulting from the antimicrobial action of that gas.

This purge can be accomplished by first removing the gas present in the enclosure using the vacuum source 12. Next, a sterile purge gas can be introduced. Once sufficient purge gas has been introduced into enclosure 10, a vacuum is again drawn on the enclosure, removing the purge gas and residuals from the sterilized enclosure. This step can be repeated if necessary, or desired, to provide several evacuation and flushing cycles.

Whether or not a purge step is required depends upon the end use to which the sterilized enclosure is to be put. Only if removal of hydrogen peroxide residuals is desired is an evacuation-and-flush purge cycle necessary. The exact number of evacuation-and-flushing cycles to be used in any particular application is determined to a large extent by the volume of the sterilized enclosure as well as by the intended end use of the sterilized container. The larger the volume the greater the number of evacuation-and-flushing cycles needed for the removal of residual material from the sterilization process. If the enclosure 10 is to be filled after sterilization with an intravenous fluid or with a dialysate for peritoneal dialysis, four to five such cycles are preferred.

The extent of evacuation using the vacuum source 12 during any given cycle is also a factor in determining the number of evacuation and flushing cycles. Evacuation of the enclosure 10 during any cycle is to a residual pressure of no more than about 50 mmHg. The number of evacuation-and-flushing cycles, in any given instance, can be reduced by evacuation to a relatively lower residual pressure of about 20 mmHg, or lower, for example.

Following either the evacuation of the sterilizing gas from the enclosure 10 or the optional evacuation and flushing cycle or cycles, the sterilized enclosure 10 is filled with the desired flowable material contents which can be a fluid or a solid, particulate or otherwise, and then sealed prior to disconnecting the sterilization system from the enclosure. Sealing can be accomplished by either heat sealing the enclosure or by any other appropriate method which provides a hermetic seal, such as ultrasonic bonding or the like.

Alternatively, if the use for the sterilized enclosure is intended to be that of a container for specimens, the sterilized container is sealed from its surroundings immediately after sterilization and again prior to disconnecting the sterilization system from the enclosure. Similarly, if the sterilized enclosure 10 is an antechamber or a connector for a container to be filled, it can be sterilized before further operations are undertaken.

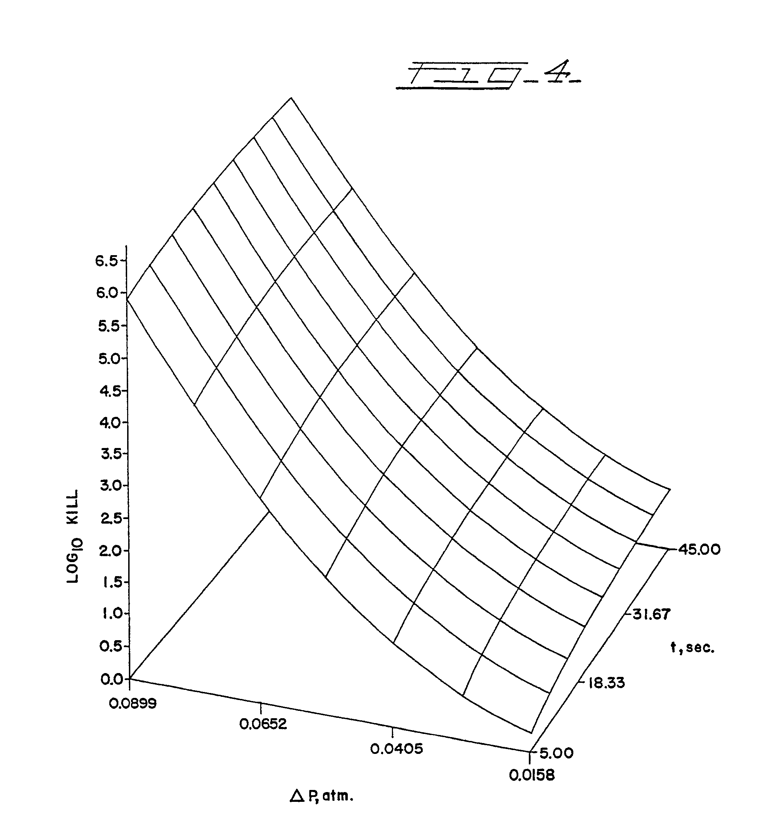

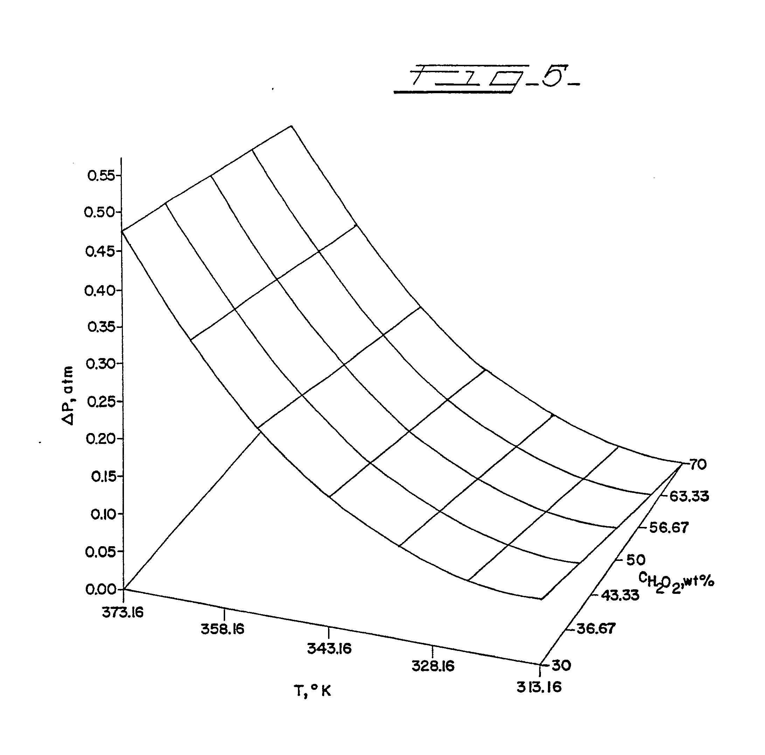

A connector 24, illustrated in FIGURES 2 and 3, is provided for facilitating the practice of this invention. Connector 24 is a hollow tube that defines a confined fluid flow communication passageway between the various systems for vacuum, sterilization gas, purge gas and fill and the enclosure to be sterilized 10. The connector 24 has an open end 25 that defines an access cavity, a septum 28 at an intermediate position within the hollow tube and which is pierceable by a nozzle 22 (FIGURE 3) which, in turn, connects the container 40 with the sterilization apparatus, and dispensing end 27. The connector 24 is permanently attached to container 40 about dispensing end 27 isolating the connector 24 and container 40 from the atmosphere.

FIGURE 3 illustrates use of the connector 24 when secured in a gas-tight manner to the inlet 26 of the container 40 which can be a sterile container or a container to be sterilized. A seal is formed at 32 when the distal, tapered end of mandrel 30 with the nozzle 22 therein mates with or is removably received within open end 25 of the connector 24 isolating the connector 24 and bag-type container 40 from the environment. The pierceable septum 28 of connector 24 is shown penetrated by the nozzle 22 in phantom which in turn, communicates with a vacuum source such as 12, sterilization gas source such as 16, and purge gas source such as 18. The nozzle also provides fluid communication for the enclosure 40 with a product source such as 19, if it is desired to fill the container 40 as well.

A flexible membrane 31 forms a seal between the exterior portion of nozzle 22 and the interior portion of the distal, tapered end of mandrel 30 to ensure no access to the container 40 other than through nozzle 22. The membrane 31 is sufficiently flexible so that the seal is maintained even when the nozzle is in its fully extended position.

A sterilization antechamber 29 is defined by mandrel 30 in cooperation with connector open end 25 when the distal, tapered end of mandrel 30 is received therewithin as shown in FIGURE 3. The tapered periphery of mandrel 30 engages the distal end portion of open end 25 to provide a seal 32. Initially nozzle 22 is positioned above septum 28 while antechamber 29 is sterilized as described hereinabove. Antechamber 29 can be sterilized, along with that portion of nozzle 22 that is situated within antechamber 29, with or without subsequent evacuation-and-flush purge cycles, as required for the contemplated filling operation for container 40.

If the container 40 has been presterilized, next the sterilized portion of nozzle 22 pierces septum 28, container 40 is filled with the desired contents, and thereafter sealed at about plane 20 to ensure continued sterility of the container and its contents. Thereafter nozzle 22 and mandrel 30 are separated from connector 24 and positioned for a subsequent sterilization and filling procedure.

Sealing of the container 40 at about plane 20 can be effected in any convenient manner as discussed hereinabove with reference to enclosure 10.

On the other hand, if container 40 has not been presterilized, it can be now sterilized by first penetrating septum 28 with nozzle 22 and then repeating the sterilization procedure carried out for the antechamber 29. The sterilization conditions and the purge cycles can be the same or different, depending upon the relative volumes of the antechamber 29 and the container 40.

The sterilization of antechamber 29 can be optional if container 40 has not been presterilized, if the pierced septum 28 provides an adequate seal with nozzle 22, because in such a case the interior of container 40 and the intruding portion of nozzle 22 are sterilized simultaneously as container 40 is sterilized.

The present sterilization method permits the utilization of aqueous hydrogen peroxide solutions containing a relatively high concentration of hydrogen peroxide. Hydrogen peroxide solutions with up to 70 weight percent of hydrogen peroxide can be used in the method disclosed herein. Higher hydrogen peroxide concentrations are undesirable from the standpoint of safety or oxidizing propensity. Highly concentrated aqueous hydrogen peroxide solutions may cause flesh burns upon contact with the skin or the eyes or vapor inhalation, and also may oxidize surfaces in contact therewith.

For purposes of this invention, aqueous solutions having a hydrogen peroxide concentration in the range of about 40 weight percent to about 60 weight percent are preferred. Particularly preferred are aqueous solutions containing about 50 weight percent of hydrogen peroxide.

The present process is well suited for the sterilization not only of containers destined for a sterile filling operation but also for sterilization of relatively large enclosures such as lyophilization chambers, or the like, connectors on previously filled containers such as dialysate bags for continuous ambulatory peritoneal dialysis, bags containing intravenous fluids, or the like, as well as medical instruments, kit and like devices.

If the enclosure to be sterilized is a flexible bag, vacuum can be applied to the outside of the bag to keep the bag from collapsing during each evacuation step.

The inter-relationship of residual pressure change and sterilization time for a desired degree of Bacillus subtilis niger spore kill when using a 50-weight percent aqueous hydrogen peroxide solution in the process of the present invention is shown in FIGURE 4. It will be seen that the sterilization time is a relatively minor factor vis-a-vis the degree of kill, and that the target degree of kill can be readily monitored during a sterilization procedure by noting the change in residual pressure as an aliquot of the sterilizing gas is introduced into the enclosure to be sterilized. The mathematical correlation for the graphical presentation depicted in FIGURE 4 is

$$\log_{10} \text{kill} = 715.833647 \, \Delta p + 0.72408 \log_{10} t - 0.39996$$

Where $\Delta p$ denotes the change in residual pressure, in atmospheres, upon addition of 50-weight percent aqueous hydrogen peroxide solution and t denotes the time, in seconds, the enclosure or product to be sterilized is exposed to the sterilizing gas.

FIGURE 5 illustrates the interrelationship between change in the aforementioned residual pressure on one hand and temperature as well as hydrogen peroxide concentration in the introduced sterilizing gas on the other. For the graphical presentation of FIGURE 5 the mathematical correlation is

$$\Delta p = 4.8 \times 10^{-5} \, T^2 + 2.422867 \times 10^{-10} \, T^4 - 0.214183 \, C_{H_2O_2} + 2.52593$$

where $\Delta p$ is as defined hereinabove with respect to FIGURE 4, T is temperature expressed in degrees Kelvin, and

$$C_{H_2O_2}$$

is the concentration of hydrogen peroxide in a $H_2O_2/H_2O$ solution, expressed as weight percent based on total weight of the solution.

The above discussion is intended by way of example only and is not intended to limit the invention in any way except in the spirit and scope of the appended claims.

## Claims

1. A method for sterilizing an enclosure to a desired probability of bacterial spore survival which comprises the steps of:

    isolating the enclosure from its surroundings;

    evacuating the isolated enclosure by reducing the pressure therein to no more than about 50 millimeters of mercury;

    introducing into the evacuated enclosure in a single step an aliquot of a dry sterilizing gas containing a sporicidal amount of gaseous hydrogen peroxide;

    retaining the introduced aliquot of the sterilizing gas within the enclosure while maintaining non-condensing conditions in the enclosure for the sterilizing gas present therein for a time period sufficient to kill the bacterial spore population present in the enclosure by the desired orders of magnitude, thereby providing a sterilized enclosure; and

    thereafter sealing the sterilized enclosure.

2. The method of claim 1 wherein the concentration of hydrogen peroxide in the sterilizing gas is about 1.5 mg/liter to about 150 mg/liter.

3. The method of claim 1 wherein the concentration of hydrogen peroxide in the sterilizing gas is about 5 mg/liter to about 100 mg/liter.

4. A sterile fill method for filling a sterile container with a flowable material which comprises the steps of:

    providing a closed sterile container that defines a hollow body portion for receiving the flowable

6

material and is equipped with an access connector constituted by a hollow tube defining a confined flow passageway that communicates with said hollow body portion and a pierceable septum sealing said passageway at an intermediate position within the tube and together with said tube defining an access cavity;

isolating said access cavity from the surroundings by sealably seating within the access cavity a fill nozzle and thereby defining a sterilization antechamber;

evacuating the sterilization antechamber through the fill nozzle to a residual pressure of no more than about 50 millimeters of mercury;

introducing into the sterilization antechamber, under noncondensing conditions, a sterilizing gas containing a sporicidal amount of gaseous hydrogen peroxide and maintaining said noncondensing conditions within the sterilization antechamber for a time period sufficient to reduce the viable bacterial spore population present to a predetermined level;

thereafter advancing the fill nozzle so as to pierce the septum, and penetrating therethrough to provide communication with the hollow container body portion;

dispensing the flowable material into the container body portion;

sealing the container body portion containing the dispensed material while the fill nozzle penetrates the pierced septum; and

withdrawing the fill nozzle from the access cavity.

5. The method of claim 4 wherein the concentration of hydrogen peroxide in the sterilizing gas is about 1.5 mg/liter to about 150 mg/liter.

6. The method of claim 4 wherein the concentration of hydrogen peroxide in the sterilizing gas is about 5 mg/liter to about 100 mg/liter.

7. A method for sterilizing an access to a filled container which comprises the steps of:

providing a filled container having a closed connector that defines a hollow body portion for receiving a flowable contents and is equipped with an access connector constituted by a hollow tube defining a confined flow passageway that communicates with said hollow body portion and a pierceable septum sealing said passageway at an intermediate position within the tube and together with said tube defining an access cavity;

isolating said access cavity from the surroundings by sealably seating within said access cavity a fill nozzle and thereby defining a sterilization antechamber;

evacuating the sterilization antechamber through the fill nozzle to a residual pressure of no more than about 50 millimeters of mercury; and

introducing into the sterilization antechamber, under noncondensing conditions, a dry sterilizing gas containing a sporicidal amount of gaseous hydrogen peroxide and maintaining said noncondensing conditions within the sterilization antechamber for a time period sufficient to reduce the viable bacterial spore population present to a predetermined level.

8. The method of claim 7 wherein the concentration of the hydrogen peroxide in the sterilizing gas is about 1.5 mg/liter to about 150 mg/liter.

9. The method of claim 7 wherein the concentration of hydrogen peroxide in the sterilizing gas is about 5 mg/liter to about 100 mg/liter.

10. The method of claim 7 wherein the amount of hydrogen peroxide in the gas is no more than about 70 weight percent, based on the weight of the gas.

EP 0 452 780 A2

FIG-1.

FIG-2.

FIG-3.

8

_FIG - 4_

_FIG. 5_